(19) Europäisches Patentamt European Patent Office Office européen des brevets

(11) **EP 1 829 438 B1**

(12) **FASCICULE DE BREVET EUROPEEN**

(45) Date de publication et mention de la délivrance du brevet:
**17.12.2014 Bulletin 2014/51**

(51) Int Cl.:
***H05H 1/46*** *(2006.01)*    ***H01J 37/32*** *(2006.01)*
***H05B 6/68*** *(2006.01)*

(21) Numéro de dépôt: **05850568.6**

(22) Date de dépôt: **21.12.2005**

(86) Numéro de dépôt international:
**PCT/FR2005/003223**

(87) Numéro de publication internationale:
**WO 2006/070107 (06.07.2006 Gazette 2006/27)**

(54) **DISPOSITIF DE STERILISATION PAR PLASMA GAZEUX**

VORRICHTUNG ZUR STERILISATION VON GASFÖRMIGEM PLASMA

DEVICE FOR GASEOUS PLASMA STERILIZATION

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **23.12.2004 FR 0413818**

(43) Date de publication de la demande:
**05.09.2007 Bulletin 2007/36**

(73) Titulaire: **Satelec-Société pour la conception des applications des Techniques Electroniques F-33700 Merignac (FR)**

(72) Inventeurs:
• **REGERE, Paul 33200 Bordeaux (FR)**
• **RICARD, André 31400 Toulouse (FR)**

• **COUSTY, Sarah 31000 Toulouse (FR)**

(74) Mandataire: **Desormiere, Pierre-Louis et al Cabinet Beau de Loménie 158, rue de l'Université 75340 Paris Cedex 07 (FR)**

(56) Documents cités:
**EP-A- 0 424 365    US-A- 4 317 976
US-A- 4 849 595    US-A- 5 325 020
US-B1- 6 445 596**

• **PATENT ABSTRACTS OF JAPAN vol. 018, no. 372 (E-1577), 13 juillet 1994 (1994-07-13) & JP 06 104079 A (SANYO ELECTRIC CO LTD), 15 avril 1994 (1994-04-15)**

EP 1 829 438 B1

## Description

La présente invention concerne un dispositif de stérilisation notamment destiné à des instruments médicaux du type faisant appel à un plasma gazeux.

On rappellera que dans les techniques de stérilisation faisant appel à un tel plasma, on utilise un gaz, n'ayant pas de lui-même des propriétés bactéricides, que l'on soumet à un champ électrique suffisamment élevé pour provoquer son ionisation et la dissociation de ses molécules. Le gaz produit en aval du plasma, désigné "gaz de post-décharge", possède des propriétés stérilisantes. Ce gaz est admis dans une chambre de traitement où il exerce son action bactéricide sur les instruments à stériliser.

On a proposé dans l'état antérieur de la technique deux voies principales permettant de produire des champs électriques dont l'intensité soit suffisante pour provoquer l'émission du plasma, à savoir les courants haute fréquence (courants HF), et les micro-ondes.

La technique des courants haute fréquence a l'inconvénient de mettre en oeuvre des électrodes qui s'usent et qui ne permettent pas d'obtenir une bonne stabilité du dispositif si bien que ce dernier doit être réajusté en permanence.

La technique des micro-ondes ne présente pas ces inconvénients mais elle n'est pas exempte néanmoins de certaines contraintes, notamment au niveau de la durée de vie et de la stabilité en fréquence du magnétron générant les micro-ondes.

On sait en effet qu'une source de micro-ondes est constituée d'un magnétron qui délivre son énergie dans un guide d'onde qui transmet celle-ci à une cavité résonante absorbante d'énergie, dans laquelle on souhaite effectuer un certain travail. Cette cavité absorbe ainsi une certaine partie de l'énergie émise, et une partie de l'énergie restante est réfléchie vers le magnétron. Or la durée de vie de celui-ci est directement liée à cette puissance réfléchie. En effet si cette dernière est trop importante elle engendre une montée en température du magnétron qui peut aller jusqu'au claquage du filament de celui-ci.

Or dans le cas où, sur le plan industriel, on souhaite produire un plasma gazeux, notamment afin d'utiliser le gaz post-décharge issu de celui-ci afin de réaliser la stérilisation d'instruments chirurgicaux, il importe que le magnétron ait une durée de vie importante compatible avec celle communément admise dans le domaine des industries médicales. Or, par définition, la puissance absorbée dans la cavité résonante est essentiellement variable puisqu'elle dépend de la masse des instruments à stériliser. En conséquence il est donc important que le magnétron puisse fonctionner avec une puissance réfléchie correspondant à sa puissance totale (ce qui correspond à une cavité résonante quasiment vide) et ceci un nombre important de fois sans subir de dégâts irréversibles.

Par ailleurs on sait également que l'excitation d'un plasma gazeux par les micro-ondes nécessite une fréquence rigoureusement stable car la cavité résonante a un coefficient de qualité très pointu, si bien que si la fréquence se décale le dispositif se désaccorde et la puissance transmise au plasma gazeux n'est plus dès lors suffisante pour assurer son entretien.

Le document US 5 325 020 décrit un dispositif de stérilisation par plasma comprenant un magnétron pour la ionisation d'un gaz.

La présente invention a pour but de proposer un générateur de micro-ondes destiné à la production d'un plasma gazeux qui pallie à ces inconvénients en assurant une excellente stabilité de fonctionnement et une durée de vie optimale de son magnétron.

La présente invention a ainsi pour objet un dispositif de production d'un plasma gazeux par ionisation d'un gaz au moyen d'une source de micro-ondes de puissance nominale déterminée, comprenant un magnétron, recevant son énergie électrique d'un circuit d'alimentation, caractérisé en ce que le circuit d'alimentation comprend des moyens pour limiter la puissance délivrée au magnétron à une valeur égale au plus au quart de la puissance nominale du magnétron. Préférentiellement cette puissance sera comprise entre le dixième et le quart de la puissance nominale du magnétron.

Préférentiellement également la puissance délivrée par le circuit d'alimentation du magnétron sera au plus égale au quart du produit de la puissance nominale du magnétron par le coefficient de réflexion de ce dernier.

Le dispositif suivant l'invention pourra comporter des moyens aptes à limiter la puissance délivrée au magnétron, qui soient tels que la température de celui-ci n'excède par 80°C.

La présente invention est particulièrement intéressante sur le plan des coûts de réalisation en ce qu'elle permet de faire appel à des circuits existant sur le marché dans le domaine ménager et qui, en raison de leur fabrication en très grandes séries, sont d'un prix de revient particulièrement compétitif. Un inconvénient de tels circuits lorsque l'on souhaite les utiliser dans certains domaines tels que le domaine médical de la stérilisation est que, d'une part, ils sont dotés d'une puissance importante de l'ordre de 800 W alors que dans le domaine de la stérilisation la puissance absorbable par la cavité de traitement est de l'ordre de seulement 100W et que, d'autre part, ils sont d'une faible fiabilité.

En ce qui concerne l'excès de puissance on comprend bien entendu qu'il n'est pas envisageable d'utiliser tel quel un tel circuit puisque la puissance réfléchie qui serait alors de l'ordre de 700 W aurait pour effet immédiat de provoquer un échauffement du magnétron entraînant sa destruction.

Pour utiliser de tels circuits il est donc nécessaire de limiter leur puissance. Par ailleurs on sait que les magnétrons doivent pouvoir disposer pour s'amorcer d'un pic de tension d'une valeur relativement élevée, de l'ordre de 3 à 4 kv.

Cette limitation de puissance doit donc s'effectuer sans porter atteinte de façon notable au pic de tension nécessaire au déclenchement du magnétron.

Suivant l'invention on limitera la puissance d'alimentation du magnétron ce qui aura pour effet de limiter l'énergie réfléchie vers celui-ci, et cette limitation interviendra sans réduire la tension de pic nécessaire à l'amorçage.

Une façon particulièrement intéressante de diminuer la puissance électrique fournie au magnétron tout en maintenant ladite tension de pic à une valeur suffisante est d'utiliser un doubleur de tension mettant en oeuvre une diode et un condensateur disposés en série aux bornes de l'enroulement secondaire et d'utiliser un condensateur d'une valeur suffisamment faible pour faire chuter la tension. On a constaté que dans ces conditions on diminuait suffisamment la puissance fournie par le magnétron pour lui assurer une fiabilité suffisante tout en préservant sa tension de pic d'amorçage.

On sait que les magnétrons sont caractérisés par un coefficient qui caractérise leur puissance maximale admissible et qui est le Rapport d'Ondes Stationnaires (ROS) :

$$ROS = \;1+r/1-r$$

r étant le coefficient de réflexion qui est égal au rapport de la puissance réfléchie sur la puissance émise.

On constate ainsi que l'énergie en mesure d'être dissipée thermiquement par un magnétron est proportionnelle à sa puissance. Ainsi, sachant que le ROS moyen pour un magnétron est de l'ordre de 4 le coefficient de réflexion r correspondant est de 0,6, si bien qu'un magnétron d'une puissance nominale de 800 watts aura une puissance réfléchie admissible de 480 watts, alors que cette même valeur pour un magnétron d'une puissance nominale de 300 watts ne serait que de 180 watts.

Dans ces conditions si l'on admet que la puissance nécessaire pour une opération déterminée, telle que par exemple une stérilisation, est de 100 watts et si l'on souhaite que le dispositif soit à même de dissiper sans problème 100% de la puissance reçue (ce qui correspond sensiblement au cas d'une enceinte résonante vide) il suffira que la puissance $P_d$ délivrée au magnétron soit au plus égale à :

$$P_d \;=\; P_n \;.\; r$$

$P_n$ étant la puissance nominale du magnétron.

On remarquera que dans le cas d'une utilisation d'un magnétron de type ménager, celui-ci ayant une puissance nominale d'environ 800 watts possèdera une puissance réfléchie admissible de 480 watts, si bien qu'il sera parfaitement en mesure d'assurer avec fiabilité la production d'un plasma à fin de stérilisation nécessitant une puissance de 100 W.

On a ainsi constaté que dans ces conditions la montée en température du magnétron était très faible ce qui procurait alors une excellente stabilité en fréquence permettant la production par celui-ci d'un plasma lorsque la puissance délivrée au magnétron était comprise entre le dixième et le quart de sa puissance nominale.

On décrira ci-après à titre d'exemple non limitatif, une forme d'exécution de la présente invention, en référence au dessin annexé sur lequel :

La figure 1 est une vue schématique d'un dispositif suivant l'invention.

La figure 2 est une courbe montrant la variation de la puissance délivrée au magnétron en fonction de la valeur de la capacité du condensateur des moyens d'alimentation.

La figure 3 est une courbe représentant la variation de la tension en fonction du temps aux bornes du magnétron dans un dispositif de type de celui représenté sur la figure 1.

La figure 4 est une vue schématique d'une variante de mise en oeuvre de l'invention.

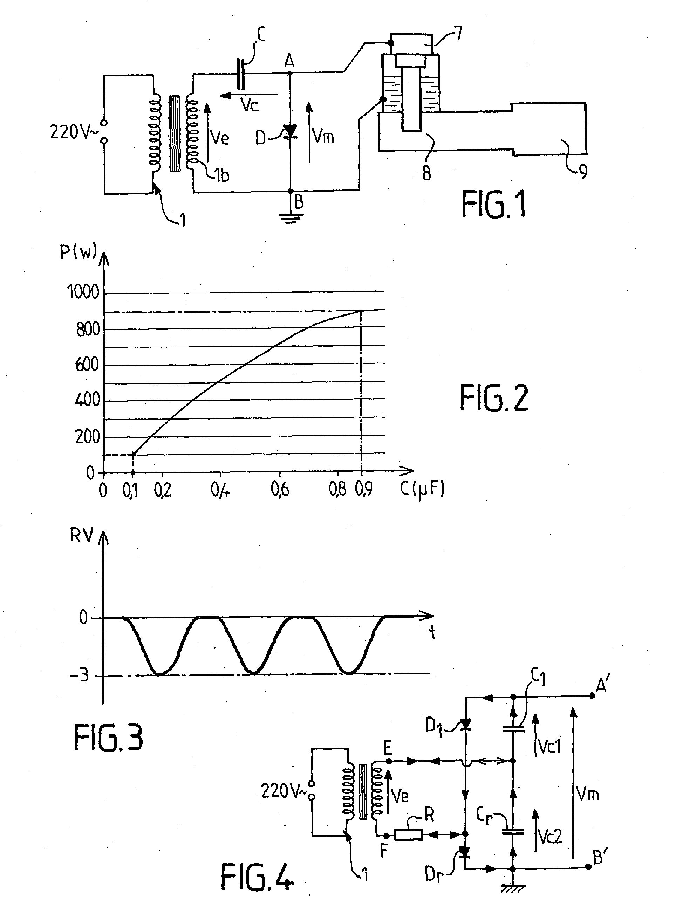

On a représenté sur la figure 1 un dispositif d'alimentation apte à fournir à un magnétron l'énergie qui lui est nécessaire pour produire un plasma gazeux. Ce plasma gazeux est notamment destiné, via son gaz de postdécharge, à assurer une fonction de stérilisation.

L'alimentation est essentiellement constituée d'un transformateur d'alimentation 1 élévateur de tension, dans un rapport d'environ 10, si bien que pour une tension d'alimentation crête à crête de 220 V on dispose au secondaire 1b de celui-ci d'une tension crête à crête d'environ 2200 V. Dans le circuit secondaire 1b on a disposé en série un condensateur C et une diode D, entre les bornes A et B de laquelle est branché un magnétron 7. Ce magnétron est réuni par un guide d'onde 8 à une cavité résonante 9.

La diode D et le condensateur C constituent un doubleur de tension permettant de multiplier par 2 la tension de sortie du transformateur 1 puisque pendant l'alternance positive le condensateur C se charge et lorsque l'alternance devient négative la tension du condensateur s'ajoute à la valeur de tension de celle-ci.

On a établi une courbe de la variation de puissance P fournie par le circuit d'alimentation au magnétron 7 en fonction de la valeur du condensateur C. On constate ainsi sur la figure 2 que la puissance P diminue avec la valeur du condensateur. Ainsi pour un condensateur C de 0,9 $\mu$F, valeur utilisée dans une alimentation classique de four à micro-ondes ménager, la puissance délivrée est environ 900 W, alors que si l'on ramène la valeur du condensateur C à 0,1 $\mu$F cette puissance chute à 100 W qui est une valeur qui correspond à celle utilisée dans le domaine particulier de la production d'un plasma gazeux en vue d'une stérilisation par son gaz de post-décharge. Ceci est particulièrement intéressant puisque, même si la puissance est totalement réfléchie, la valeur de celle-ci se trouvera en dessous de la valeur de la puissance de retour admissible qui pour un magnétron de puissance nominale de 800 W est de 480 W.

On constate ainsi que, par une simple opération de remplacement d'un composant aussi simple et aussi peu onéreux qu'un condensateur on peut adapter une alimentation bon marché du commerce et la transformer de façon qu'elle soit dès lors apte à assurer de façon fiable et efficace l'alimentation d'un magnétron destiné à un usage intensif notamment dans les domaines médical et industriel.

Par ailleurs on a représenté sur la figure 3 une courbe exprimant la variation de la tension aux bornes A et B de l'alimentation du magnétron. On constate sur celle-ci que le pic de tension en début d'alternance est bien maintenu, ce qui permet d'assurer un amorçage correct du magnétron.

On peut également suivant l'invention, ainsi que représenté sur la figure 4 réaliser une alimentation double alternance du magnétron. Dans ce montage on a réalisé une boucle comprenant deux diodes en série, à savoir une première diode D1 et une seconde diode D2, la sortie de la première étant réunie à l'entrée de la seconde et deux condensateurs C1 et C2. Une borne de sortie $\underline{E}$ du transformateur 1 est réunie entre les deux condensateurs C1 et C2, et l'autre borne de sortie F est réunie, par l'intermédiaire d'une résistance $\underline{R}$ à l'entrée de la diode D2. Le magnétron est alimenté entre la borne d'entrée A' de la première diode D1 et la borne de sortie B' de la seconde diode D2. Un tel montage cumule les deux doubleurs de tension, et la tension délivrée entre les bornes A' et B' est la somme des tensions aux bornes des condensateurs C1 et C2. En effet durant l'alternance positive le condensateur C1 se charge au travers de la diode D1. Lorsque l'alternance devient négative le condensateur C2 se charge au travers de la diode D2.

**Revendications**

1. Dispositif de stérilisation par un plasma gazeux obtenu par ionisation d'un gaz, ledit dispositif comprenant une source de micro-ondes de puissance nominale ($P_n$) déterminée, comprenant un magnétron (7), un circuit d'alimentation, une cavité résonnante (9) et un guide d'onde (8), le guide d'onde (8) réunissant le magnétron (7) à la cavité résonnante (9), et le magnétron (7) recevant son énergie électrique du circuit d'alimentation, **caractérisé en ce que** le magnétron (7) présente une puissance nominale d'environ 800 W et nécessite un pic de tension de l'ordre de 3 à 4 kV pour amorcer et **en ce que** le circuit d'alimentation comprend des moyens pour limiter la puissance ($P_d$) délivrée au magnétron (7) à une valeur égale au plus au quart de la puissance nominale ($P_n$) du magnétron (7).

2. Dispositif suivant la revendication 1 **caractérisé en ce que** la puissance ($P_d$) délivrée par le circuit d'alimentation au magnétron (7) est comprise entre le dixième et le quart de la puissance nominale ($P_n$) du magnétron.

3. Dispositif suivant la revendication 1 **caractérisé en ce que** la puissance ($P_d$) délivrée par le circuit d'alimentation au magnétron est au plus égale au quart du produit de la puissance nominale ($P_n$) du magnétron par le coefficient de réflexion (r) de ce dernier.

4. Dispositif suivant l'une des revendications précédentes **caractérisé en ce qu'**il comporte des moyens aptes à limiter la puissance ($P_d$) délivrée au magnétron, tels que la température de celui-ci n'excède pas 80°C.

5. Dispositif suivant l'une des revendications précédentes **caractérisé en ce que** les moyens d'alimentation du magnétron comportent des moyens doubleurs de tension.

6. Dispositif suivant la revendication 5 **caractérisé en ce que** les moyens doubleurs de tension sont constitués d'une diode (D) et d'un condensateur (C) disposés en série aux bornes d'un transformateur d'alimentation (1), le magnétron (7) étant alimenté aux bornes de la diode (D).

7. Dispositif suivant la revendication 6 **caractérisé en ce que** la valeur du condensateur (C) est voisine de 0,1 μF.

8. Dispositif suivant l'une des revendications 1 à 5 **caractérisé en ce que** les moyens doubleurs de tension sont constitués d'une boucle formée de deux diodes en série, à savoir une première diode (D1) et une seconde diode (D2), la sortie de la première étant réunie à l'entrée de la seconde, et de deux condensateurs (C1) et (C2), une borne de sortie (E) du transformateur (1) étant réunie entre les deux condensateurs (C1,C2) et l'autre borne de sortie (F) étant réunie, par l'intermédiaire d'une résistance (R), à l'entrée de la seconde diode (D2), le magnétron (7) étant alimenté entre la borne d'entrée (A') de la première diode (D1) et la borne de sortie (B') de la seconde diode (D2).

**Patentansprüche**

1. Vorrichtung zur Sterilisation mittels eines gasförmigen Plasmas, das durch Ionisierung eines Gases erhalten wird, wobei die Vorrichtung eine Mikrowel-

lenquelle mit bestimmter Nennleistung ($P_n$) umfasst, mit einem Magnetron (7), einem Versorgungskreis, einem Resonanzraum (9) und einem Wellenleiter (8), wobei der Wellenleiter (8) das Magnetron (7) mit dem Resonanzraum (9) verbindet und wobei das Magnetron (7) seine elektrische Energie von dem Versorgungskreis erhält, **dadurch gekennzeichnet, dass** das Magnetron (7) eine Nennleistung von etwa 800 W aufweist und einen Spannungspeak in der Größenordnung von 3 bis 4 kV benötigt, um in Gang zu kommen, und dass der Versorgungskreis Mittel umfasst, um die an das Magnetron (7) gelieferte Leistung ($P_d$) auf einen Wert zu begrenzen, der höchstens gleich dem Viertel der Nennleistung ($P_n$) des Magnetrons (7) ist.

2. Vorrichtung nach Anspruch 1, **dadurch gekennzeichnet, dass** die durch den Versorgungskreis an das Magnetron (7) gelieferte Leistung ($P_d$) zwischen dem Zehntel und dem Viertel der Nennleistung ($P_n$) des Magnetrons liegt.

3. Vorrichtung nach Anspruch 1, **dadurch gekennzeichnet, dass** die durch den Versorgungskreis an das Magnetron gelieferte Leistung ($P_d$) höchstens gleich dem Viertel des Produktes aus der Nennleistung ($P_n$) des Magnetrons und dem Reflexionskoeffizienten (r) dieses letzteren ist.

4. Vorrichtung nach einem der vorhergehenden Ansprüche, **dadurch gekennzeichnet, dass** sie Mittel umfasst, die geeignet sind, die an das Magnetron gelieferte Leistung ($P_d$) zu begrenzen, die derart sind, dass die Temperatur dessen 80 °C nicht überschreitet.

5. Vorrichtung nach einem der vorhergehenden Ansprüche, **dadurch gekennzeichnet, dass** die Versorgungsmittel des Magnetrons Spannungsverdopplermittel umfassen.

6. Vorrichtung nach Anspruch 5, **dadurch gekennzeichnet, dass** die Spannungsverdopplermittel von einer Diode (D) und von einem Kondensator (C) gebildet sind, die an den Anschlüssen eines Speisetransformators (1) in Reihe angeordnet sind, wobei das Magnetron (7) an den Anschlüssen der Diode (D) versorgt wird.

7. Vorrichtung nach Anspruch 6, **dadurch gekennzeichnet, dass** der Wert des Kondensators (C) nahe 0,1 $\mu$F liegt.

8. Vorrichtung nach einem der Ansprüche 1 bis 5, **dadurch gekennzeichnet, dass** die Spannungsverdopplermittel von einer Schleife gebildet sind, welche von zwei Dioden in Reihe, nämlich einer ersten Diode (D1) und einer zweiten Diode (D2), wobei der Ausgang der ersten mit dem Eingang der zweiten verbunden ist, sowie von zwei Kondensatoren (C1) und (C2) gebildet ist, wobei ein Ausgangsanschluss (E) des Transformators (1) zwischen den beiden Kondensatoren (C1, C2) verbunden ist und wobei der andere Ausgangsanschluss (F) mittels eines Widerstandes (R) mit dem Eingang der zweiten Diode (D2) verbunden ist, wobei das Magnetron (7) zwischen dem Eingangsanschluss (A') der ersten Diode (D1) und dem Ausgangsanschluss (B') der zweiten Diode (D2) versorgt wird.

**Claims**

1. Device for sterilization using a plasma gas by ionisation of a gas, said device comprising a microwave source of determined nominal power (Pn), comprising a magnetron (7), a power supply circuit, a resonant cavity (9) and a waveguide (8), the waveguide (8) connecting the magnetron (7) to the resonant cavity (9) and the magnetron (7) receiving its electric energy from the power supply circuit, **characterized in that** the magnetron (7) has a nominal power ($P_n$) of about 800 W and requiring a peak voltage of about 3-4 KV to initiate and **in that** the supply circuit comprises means for limiting the power (Pd) delivered by the supply circuit to the magnetron (7) to a value no more than one quarter of the nominal power ($P_n$) of the magnetron (7).

2. Device as in claim 1, **characterized in that** the power ($P_d$) delivered by the supply circuit to the magnetron (7) lies between one tenth and one quarter of the nominal power ($P_n$) of the magnetron.

3. Device as in claim 1, **characterized in that** the power ($P_d$) delivered by the supply circuit to the magnetron is no more than one quarter of the product of the nominal power ($P_n$) of the magnetron multiplied by its reflection coefficient (r).

4. Device as in any of the preceding claims, **characterized in that** it comprises means able to limit the power (Pd) delivered to the magnetron, such that its temperature does not exceed 80°C.

5. Device as in any of the preceding claims, **characterized in that** the magnetron supply means comprise voltage doubler means.

6. Device as in claim 5, **characterized in that** the voltage doubler means consist of a diode (D) and a capacitor (C) arranged in series at the terminals of a supply transformer (1), the magnetron (7) being supplied at the terminals of the diode (D).

7. Device as in claim 6, **characterized in that** the value of the capacitor (C) is close to 0.1 $\mu$F.

8. Device as in any of claims 1 to 5, **characterized in that** the voltage doubler means consist of a loop formed of two diodes in series, namely a first diode (D1) and a second diode (D2), the output of the first being joined to the input of the second, and of two capacitors (C1) and (C2), one output terminal (E) of transformer (1) being joined between the two capacitors (C1,C2) and the other output terminal (F) being joined, via a résistance (R), to the input of the second diode (D2), the magnetron (7) being supplied between the input terminal (A') of the first diode (D1) and the output terminal (B') of the second diode (D2).

FIG.1

FIG.2

FIG.3

FIG.4

**EP 1 829 438 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5325020 A